(19) Europäisches Patentamt
European Patent Office
Office européen des brevets

(11) Veröffentlichungsnummer: **0 031 088**
**A1**

(12) # EUROPÄISCHE PATENTANMELDUNG

(21) Anmeldenummer: **80107818.9**

(22) Anmeldetag: **11.12.80**

(51) Int. Cl.[3]: **C 07 D 495/04**
A 61 K 31/64
//(C07D495/04, 209/00, 333/00)

(30) Priorität: **19.12.79 DE 2951060**

(43) Veröffentlichungstag der Anmeldung:
**01.07.81 Patentblatt 81/26**

(84) Benannte Vertragsstaaten:
**AT BE CH DE FR GB IT LI NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Hitzel, Volker, Dr.**
**Kantstrasse 1b**
**D-6238 Hofheim am Taunus(DE)**

(72) Erfinder: **Weyer, Rudi, Dr.**
**Johann-Strauss-Strasse 43**
**D-6233 Kelkheim (Taunus)(DE)**

(72) Erfinder: **Geisen, Karl, Dr.**
**Jahnstrasse 43**
**D-6000 Frankfurt am Main(DE)**

(72) Erfinder: **Regitz, Günter, Dr.**
**Dachbergstrasse 68**
**D-6232 Bad Soden am Taunus(DE)**

(54) **Benzolsulfonylharnstoffe, Verfahren zu ihrer Herstellung, pharmazeutische Präparate auf Basis dieser Verbindungen sowie ihre Verwendung.**

(57) Sulfonylharnstoffe der Formel

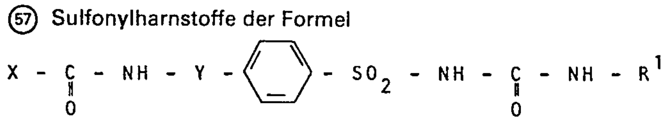

worin R[1], X und Y die angegebenen Bedeutungen haben, sowie deren physiologisch verträgliche Salze, pharmazeutische Präparate auf Basis dieser Verbindungen und ihre Verwendung bei der Behandlung des Diabetes.

**EP 0 031 088 A1**

HOECHST AKTIENGESELLSCHAFT HOE 79/F 342 Dr.D/Pa

Benzolsulfonylharnstoffe, Verfahren zu ihrer Herstellung, pharmazeutische Präparate auf Basis dieser Verbindungen sowie ihre Verwendung

Die Erfindung betrifft Sulfonylharnstoffe der Formel

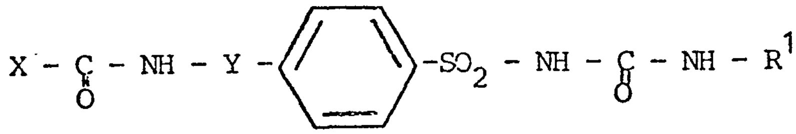

die als Substanz oder in Form ihrer Salze blutzuckersenkende Eigenschaften besitzen und sich durch starke und langanhaltende Senkung des Blutzuckerspiegels auszeichnen.

In der Formel bedeuten

X

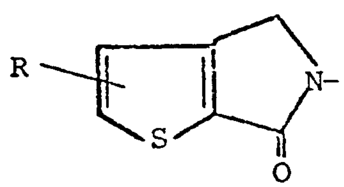
,
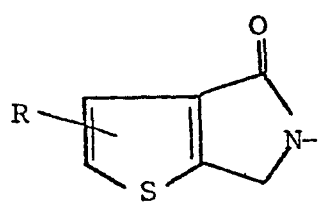
oder
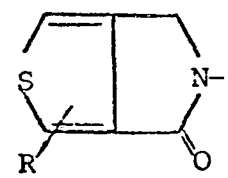

worin R Wasserstoff oder Halogen ist

Y Alkylen mit 2 - 3 C-Atomen,

$R^1$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl, mit jeweils 5 - 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl.

X bedeutet vorzugsweise einen Rest der Formel 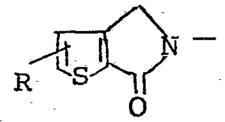

In den allgemeinen Formeln bedeutet R vorzugsweise Wasserstoff. Y bedeutet vorzugsweise $-CH_2-CH_2-$ und $-\underset{CH_3}{\underset{|}{CH}}-CH_2-$, wobei die $-CH_2-CH_2-$Gruppe besonders bevorzugt ist. $R^1$ ist vorzugsweise Cyclopentyl, Cyclohexyl, Methylcyclohexyl, 3-Methylcyclopentylmethyl, besonders bevorzugt ist Cyclohexyl und 3-Methylcyclopentylmethyl. Als bicyclische Reste für $R^1$ kommen in Frage: Bicyclo [2.2.1]heptyl, Bicyclo-[2.2.1]heptyl-methyl sowie die entsprechenden ungesättigten Reste und der Bicyclo [2.2.2]octylrest.

Die Erfindung betrifft ferner Verfahren zur Herstellung dieser Sulfonylharnstoffe, pharmazeutische Präparate, die diese enthalten oder aus ihnen bestehen sowie deren Verwendung zur Behandlung des Diabates.

Die Verfahren zur Herstellung sind dadurch gekennzeichnet, daß man

a) mit der Gruppe

$$X - CO - NH - Y -$$

in 4-Stellung substituierte Benzolsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

X - CO - NH - Y - 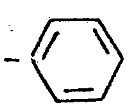 $-SO_2 - NH_2$

oder deren Salzen mit $R^1$-substituierten Isocyanaten, Carbaminsäureestern, Thiolcarbaminsäureestern, Carbaminsäurehalogenide oder Harnstoffen umsetzt,

b) entsprechend substituierte Benzolsulfonyl-isohanrstoffäther, -isothioharnstoffäther, -parabansäuren oder -halogenameisensäureamidine spaltet,

c) in

$$X - CO - NH - Y -$$

substituierten Benzolsulfonylthioharnstoffen das Schwefelatom durch Sauerstoff ersetzt,

d) in Benzolsulfonylharnstoffe der Formel

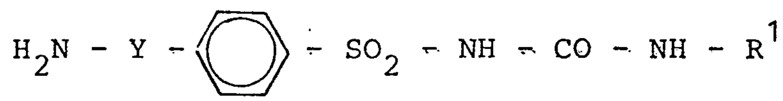

gegebenenfalls stufenweise den Rest

$$X - CO -$$

einführt, und die Reaktionsprodukte gegebenenfalls zur Salzbildung mit alkalischen Mitteln behandelt.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die N-$R^1$-substituierten Carbaminsäureester bzw. die entsprechenden Thiolcarbaminsäureester.

Als Carbaminsäurehalogenide eignen sich in erster Linie die Chloride.

Die als Ausgangsstoffe des Verfahrens infrage kommenden Benzolsulfonylharnstoffe können an der der Solfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden.

Neben alkyl-, aryl-, acyl- oder heterocyclisch substituierten Benzolsulfonylharnstoffen kann man auch Benzolsulfonylcarbamoylimidazole und ähnliche Verbindungen oder Bisbenzolsulfonylharnstoffe, die an einem der Stickstoffatome noch einen weiteren Substituenten z.B. Methyl, tragen können, verwenden. Man kann beispielsweise derartige Bis-(benzolsulfonyl)-harnstoffe oder auch N-Benzolsulfonyl-N'-acylharnstoffe mit $R^1$-substituierten Aminen behandeln und die erhaltenen Salze auf erhöhte Temperaturen, insbesondere solche oberhalb $100^oC$, erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

$$X - CO - NH - Y -$$

in 4-Stellung substituierten Benzolsulfonamiden umzusetzen. Als solche Ausgangsstoffe kommen beispielsweise infrage N-Cyclohexyl-harnstoff, die entsprechenden N´-Acetyl, N´-Nitro, N´-Cyclohexyl, N´,N´-Diphenyl- (wobei die beiden Phenylreste auch substituiert sowie direkt oder auch über ein Brückenglied wie $-CH_2-$, -NH-, -O- oder -S-miteinander verbunden sein können), N´-Methyl-N´-phenyl-, N´,N´-Dicyclohexylharnstoffe sowie Cyclohexyl-carbamoylimidazole,-pyrazole oder -triazole sowie solche der genannten Verbindungen, die anstelle des Cyclohexyls einen anderen im Bereich der Definition für $R^1$ liegenden Substituenten tragen.

Die Spaltung der als Ausgangsstoffe genannten Benzolsulfonylparabansäuren, -isoharnstoffäther, -isothioharnstoffäther oder -halogenameisensäureamidine erfolgt zweckmäßig durch alkalische Hydrolyse. Isoharnstoffäther könne auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagerung von Wasser in die Benzolsulfonylharnstoffe überführt werden.

Die Acylierung der Sulfonylharnstoffe gemäß Verfahren d) kann mit reaktiven Derivaten der Säure

X - COOH

wie beispielsweise Halogeniden und Urethanen erfolgen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Synthese der Oxo-thieno-pyrrol-Verbindungen erfolgt nach bekannten Methoden zur Herstellung von Lactamen.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man eine Substanz aus einem kristallinen (Alkali-)-Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignen sich daher als Arzneimittel, insbesondere als Antidiabetika.

Die blutzuckersenkende Wirkung der erfindungsgemäßen Benzolsulfonylharnstoffe kann dadurch festgestellt werden, daß man sie als freie Verbindungen oder in Form der Natriumsalze in Dosen von 10 mg oder 2 mg/kg an normal ernährte Kaninchen verfüttert und den Blutzuckerwert nach der bekannten Methode von Hagedorn-Jensen oder mit einem Autoanalyzer über eine längere Zeitdauer ermittelt.

Die Bestimmung der blutzuckersenkenden Wirkung kann aber auch mit geringen Dosen oder nach anderen bekannten Methoden erfolgen.

Die folgenden Verbindungen I bis VI wurden in Dosierungen von 2 mg/kg Kaninchen verabreicht und die Blutzuckerwerte wurden mit einem Autoanalyzer über eine längere Zeitdauer ermittelt. Die hierbei gemessene Blutzuckersenkung ist in der nachfolgenden Tabelle in % nach 1,3,6,24 und 48 Stunden angegeben.

I N-(4-< 2-(6-Oxo-5H-thieno-< 2,3-C > -pyrrol-5-yl-carboxamido)-äthyl> -benzolsulfonyl)-N'-(4-methyl-cyclohexyl)-harnstoff

II N-(4-< 2-(6-Oxo-5H-thieno-< 2,3-C > -pyrrol-5-yl-carboxamido)-äthyl> -benzolsulfonyl)-N'-cyclohexyl-harnstoff

III N-(4-< 2-(6-Oxo-5H-thieno-< 2,3-C > -pyrrol-5-yl-carboxamido)-äthyl> -benzolsulfonyl)-N'-(3-methylcyclopentylmethyl)-harnstoff

IV N-(4-< 2-(6-Oxo-5H-thieno-< 2,3-C > -pyrrol-5-yl-carboxamido)-äthyl> -benzolsulfonyl)-N'- $\Delta^3$-cyclohexenyl-harnstoff

V N-(4-< 2-(6-Oxo-5H-thieno-< 2,3-C > -pyrrol-5-yl-carboxamido)-äthyl> -benzolsulfonyl)-N'-cyclopentylmethyl-harnstoff

VI N-(4-< 2-(6-Oxo-5H-thieno-< 2,3-C > -pyrrol-5-yl-carboxamido)-äthyl> -benzolsulfonyl)-N'-cycloheptyl-harnstoff.

T a b e l l e

| Verbindung | Blutzuckersenkung am Kaninchen nach oraler Verabreichung von 2 mg/kg in % n a c h | | | | |
|---|---|---|---|---|---|
| | 1 | 3 | 6 | 24 | 48 Stunden |
| I | 11 | 19 | 33 | 39 | 18 |
| II | 25 | 30 | 38 | 44 | 32 |
| III | 33 | 42 | 37 | 48 | 22 |
| IV | 15 | 36 | 33 | 41 | 11 |
| V | 31 | 32 | 43 | 41 | 42 |
| VI | 20 | 19 | 35 | 35 | 13 |

Die erfindungsgemäßen Acylureidoalkylbenzolsulfonylharnstoffe zeichnen sich durch eine starke und langanhaltende blutzuckersenkende Wirkung aus.

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.

Benzolsulfonylharnstoffe mit Ureidoalkylrest sind schon mehrfach beschrieben worden (DE-PS 14 43 911, DE-AS 16 70 700, DE-PS 16 18 389, DE-PS 22 38 870). Es war nicht zu erwarten, daß sich die erfindungsgemäßen Verbindungen durch die oben erwähnten günstigen Eigenschaften auszeichnen.

Die erfindungsgemäßen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische Mittel wie Alkali- oder Erdalkalihydroxyde, -carbonate oder -bicarbonate herangezogen werden. Die Präparate können neben dem Sulfonylharnstoff bzw. dessen Salzen auch noch andere Wirkstoffe enthalten.

Als medizinische Präparate kommen vorzugsweise Tabletten in Betracht, die neben den Sulfonylharnstoffen bzw. deren Salzen die üblichen Träger- und Hilfsstoffe wie Talkum, Stärke, Milchzucker oder Magnesiumstearat enthalten.

Ein Präparat, das die erfindungsgemäßen Benzolsulfonylharnstoffe als Wirkstoff enthält, z.B. eine Tablette oder ein Pulver mit oder ohne Zusätze, ist zweckmäßig

in eine geeignet dosierte Form gebracht. Als Dosis ist dabei eine solche zu wählen, die der Wirksamkeit des verwendeten Benzolsulfonylharnstoffs und dem gewünschten Effekt angepaßt ist. Zweckmäßig beträgt die Dosierung je Einheit etwa 0,1 bis 10 mg, vorzugsweise 0,5 bis 2 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können. Sie sollen jedoch nicht eine Einschränkung des Erfindungsgegenstandes darstellen.

Beispiel 1:

N-(4-<2-(6-Oxo-5H-thieno-<2,3-C>-pyrrol-5-yl-carboxamido)-äthyl>-benzolsulfonyl)-N'-(4-methyl-cyclohexyl)-harnstoff.

3,65 g 4-(2-⟨6-Oxo-5H-thieno-⟨2,3-C⟩-pyrrol-5-yl-carboxamdio⟩-äthyl)-benzolsulfonamid werden in 100 ml Aceton nach Zusatz von 2,8 g gemahlener Pottasche 4 Stunden unter Rückfluß gerührt. Nach kurzem Abkühlen tropft man eine Lösung von 1,4 g 4-Methylcyclohexylisocyanat in wenig Aceton zu und rührt weitere 4 Stunden unter Rückfluß nach. Nach dem Erkalten dampft man im Vakuum ein, nimmt den Rückstand in Wasser auf und säuert mit 2 N Salzsäure an. Der Niederschlag wird abgesaugt, aus verdünnter Ammoniak-Lösung mit 2 N Salzsäure umgefällt und nach erneutem Absaugen aus Äthanol umkristallisiert. Der so erhaltene N-(4-⟨2-(6-Oxo-5H-thieno-⟨2,3-C⟩-pyrrol-5-yl-carboxamido)-äthyl⟩-benzolsulfonyl)-N'-(4-methyl-cyclohexyl)-harnstoff schmilzt bei 230 - 231 °C.

Das als Ausgangsmaterial verwendete Sulfonamid wird auf folgendem Weg hergestellt:
3-Brommethyl-thiophen-2-carbonsäuremethylester wird über das Urotropin-Verfahren in das Hydrochlorid des 3-Aminomethyl-thiophen-2-carbonsäuremethylesters (Schmp. 202 °C) überführt. Durch 20-stündiges Rückflußkochen in Methanol/Äthanol unter Zusatz von Pottasche gelangt man zum 6-Oxo-5H-thieno-⟨2,3-C⟩-pyrrol (Schmp. 170 - 171 °C). Die Reaktion des Lactams mit 2-Phenyläthylisocyanat ergibt das 6-Oxo-5H-thieno-⟨2,3-C⟩-pyrrol-2-yl(N-2-phenyläthyl)-carboxamid (Schmp. 124 °C), aus dem durch Umsetzung mit Chlorsulfonsäure und anschließende Reaktion mit conc. Ammoniak-Lösung das 4-(2-⟨6-Oxo-5H-thieno-⟨2,3-C⟩-pyrrol-5-yl-carboxamido⟩-äthyl)-benzolsulfonamid vom Schmp. 245 °C erhalten wird.

In analoger Weise erhält man den

N-(4-⟨2-(6-Oxo-5H-thieno-⟨2,3-C⟩-pyrrol-5-yl-carboxamido)-äthyl⟩-benzolsulfonyl)-N′-butyl-harnstoff vom Schmp. 180 - 181°C (aus Äthanol)

N-(4-⟨2-(6-Oxo-5H-thieno-⟨2,3-C⟩-pyrrol-5-yl-carboxamido)-äthyl⟩-benzolsulfonyl)-N′-propyl-harnstoff vom Schmp. 186 - 187°C (aus Äthanol)

N-(4-⟨2-(6-Oxo-5H-thieno-⟨2,3-C⟩-pyrrol-5-yl-carboxamido)-äthyl⟩-benzolsulfonyl)-N′-cyclohexyl-harnstoff vom Schmp. 238 - 239°C (aus Äthanol-Dimethylformamid)

N-(4-⟨2-(6-Oxo-5H-thieno-⟨2,3-C⟩-pyrrol-5-yl-carboxamido)-äthyl⟩-benzolsulfonyl)-N′-cyclopentyl-harnstoff vom Schmp. 196 - 197°C (aus Äthanol)

N-(4-⟨2-(6-Oxo-5H-thieno-⟨2,3-C⟩-pyrrol-5-yl-carboxamido)-äthyl⟩-benzolsulfonyl)-N'-bicyclo-/2.2.1_7hept-2-yl-methyl-harnstoff vom Schmp. 227 - 228°C (aus Äthanol-Dimethylformamid)

N-(4-⟨2-(6-Oxo-5H-thieno-⟨2,3-C⟩-pyrrol-5-yl-carboxamido)-äthyl⟩-benzolsulfonyl)-N'-(3-methylcyclopentylmethyl)-harnstoff vom Schmp. 196 - 197°C (aus Äthanol)

N-(4-⟨2-(6-Oxo-5H-thieno-⟨2,3-C⟩-pyrrol-5-yl-carboxamido)-äthyl⟩-benzolsulfonyl)-N′-(3-methylcyclopentyl)-harnstoff
vom Schmp. 196 - 197°C (aus Äthanol)

N-(4-⟨2-(6-Oxo-5H-thieno-⟨2,3-C⟩-pyrrol-5-yl-carboxamido)-äthyl⟩-benzolsulfonyl)-N′-$\Delta^3$-cyclohexenyl-harnstoff
vom Schmp. 199 - 201°C (aus Äthnaol)

N-(4-⟨2-(6-Oxo-5H-thieno-⟨2,3-C⟩-pyrrol-5-yl-carboxamido)-äthyl⟩-benzolsulfonyl)-N′-hexyl-harnstoff
vom Schmp. 197 - 198°C (aus Äthanol)

N-(4-⟨2-(6-Oxo-5H-thieno-⟨2,3-C⟩-pyrrol-5-yl-carboxamido)-äthyl⟩-benzolsulfonyl)-N′-(4,4-dimethyl-cyclohexyl)-harnstoff
vom Schmp. 253 - 255°C (aus Äthanol)

N-(4-⟨2-(6-Oxo-5H-thieno-⟨2,3-C⟩-pyrrol-5-yl-carboxamido)-äthyl⟩-benzolsulfonyl)-N′-benzyl-harnstoff
vom Schmp. 206 - 208°C (aus Äthanol)

N-(4-⟨2-(6-Oxo-5H-thieno-⟨2,3-C⟩-pyrrol-5-yl-carboxamido)-äthyl⟩-benzolsulfonyl)-N′-cyclopentylmethyl-harnstoff
vom Schmp. 200 - 202°C (aus Äthanol)

N-(4-⟨2-(6-Oxo-5H-thieno-⟨2,3-C⟩-pyrrol-5-yl-carboxamido)-äthyl⟩-benzolsulfonyl)-N′-cycloheptyl-harnstoff
vom Schmp. 230 - 232°C (aus Äthanol)

N-(4-⟨2-(6-Oxo-5H-thieno-⟨2,3-C⟩-pyrrol-5-yl-carboxamido)-äthyl⟩-benzolsulfonyl)-N′-cyclooctyl-harnstoff
vom Schmp. 198 - 200°C (aus Äthanol)

Beispiel 2:

N-(4-⟨2-(6-Oxo-5H-thieno-⟨2,3-C⟩-pyrrol-5-yl-carboxamido)-äthyl⟩-benzolsulfonyl)-N′-cyclohexyl-harnstoff

5,5 g 6-Oxo-5H-thieno-⟨2,3-C⟩-pyrrol-2-yl-carbonsäure-chlorid (hergestellt aus 6-Oxo-5H-thieno-⟨2,3-C⟩-pyrrol-Na und Phosgen in Toluol) werden in eine Lösung von 11,7 g N-(4-⟨2-Aminoäthyl⟩-benzolsulfonyl)-N′-cyclohexyl-harnstoff in 100 ml Pyridin eingetragen. Nach der Zugabe erwärmt man unter Rühren vier Stunden auf 100°C, kühlt ab und gießt auf Eiswasser. Beim vorsichtigen Ansäuern mit conc. Salzsäure bildet sich ein Niederschlag, der abgesaugt und nach dem Umfällen aus verdünnter Ammoniaklösung mit verdünnter Salzsäure aus Äthanol-Dimethylformamid umkristallisiert wird. Der so erhaltene N-(4-⟨2-(6-Oxo-5H-thieno-⟨2,3-C⟩-pyrrol-5-yl-carboxamido)-äthyl⟩-benzolsulfonyl)-N′-cyclohexyl-harnstoff schmilzt bei 236 - 238°C und ist im DC-Vergleich identisch mit einer authentischen Probe.

Beispiel 3:

N-(4-⟨2-(6-Oxo-5H-thieno-⟨2,3-c⟩-pyrrol-5-yl-carboxamido)-äthyl⟩-benzolsulfonyl)-N'-cyclohexyl-harnstoff

0,75 g N-(4-⟨2-(6-Oxo-5H-thieno-⟨2,3-C⟩-pyrrol-5-yl-carboxamido)-äthyl⟩-benzolsulfonyl)-N'-cyclohexyl-thioharnstoff (Schmp. 201 - 202°C, hergestellt aus dem entsprechenden Sulfonamid mit Cyclohexylsenföl) werden in 50 ml Methanol und 50 ml Wasser nach Zusatz von 0,32 g gelbem Quecksilberoxid unter Rühren vier Stunden auf 45°C erwärmt. Nach dem Abkühlen wird das Quecksilbersulfid abfiltriert, das Filtrat eingeengt, der verbleibende Rückstand abgesaugt und aus Äthanol-Dimethylformamid umkristallisiert. Der Schmelzpunkt des so erhaltenen N-(4-⟨2-(6-Oxo-5H-thieno-⟨2,3-c⟩-pyrrol-5-yl-carboxamido)-äthyl⟩-benzolsulfonyl)-N'-cyclohexyl-harnstoffs liegt bei 235 - 237°C. Ein Mischschmelzpunkt und ein DC-Vergleich mit einer auf anderem Weg hergestellten Probe zeigten keinen Unterschied.

Beispiel 4:

N-(4-⟨2-(6-Oxo-5H-thieno-⟨2,3-C⟩-pyrrol-5-yl-carboxamido)-äthyl⟩-benzolsulfonyl)-N′-cyclohexyl-harnstoff

0,75 g N-(4-⟨2-(6-Oxo-5H-thieno-⟨2,3-C⟩-pyrrol-5-yl-carboxamido)-äthyl⟩-benzolsulfonyl)-N′-cyclohexyl-thioharnstoff werden in 50 ml wasserfreiem Methanol nach Zugabe von 0,32 g gelbem Quecksilberoxid sechs Stunden unter Rückfluß gerührt. Nach dem Abkühlen und Filtrieren engt man die Reaktionslösung ein und erhält den N-(4-⟨2-(6-Oxo-5H-thieno-⟨2,3-C⟩-pyrrol-5-yl-carboxamido)-äthyl⟩-benzolsulfonyl)-N′-cyclohexyl-isoharnstoffmethyläther vom Schmelzpunkt 183 - 184°C.

Der auf dem beschriebenen Wege erhaltene Isoharnstoffmethyläther wird in 30 ml conc. Salzsäure zwei Stunden auf dem Dampfbad erwärmt. Nach dem Verdünnen mit Wasser wird abgesaugt und aus Äthanol unter Zusatz von Dimethylformamid umkristallisiert. Der so erhaltene N-(4-⟨2-(6-Oxo-5H-thieno-⟨2,3-C⟩-pyrrol-5-yl-carboxamido)-äthyl⟩-benzolsulfonyl)-N′-cyclohexyl-harnstoff schmilzt bei 236 - 239°C.

Er zeigt im Mischschmelzpunkt mit einer nach Beispiel 1 synthetisierten Probe keine Depression und ist ebenso im DC-Vergleich identisch.

Beispiel 5:

**N-(4-⟨2-(6-Oxo-5H-thieno-⟨2,3-c⟩-pyrrol-5-yl-carboxamido)-äthyl⟩-benzolsulfonyl)-N'-cyclohexyl-harnstoff**

1,3 g N-(4-⟨2-(6-Oxo-5H-thieno-⟨2,3- c⟩-pyrrol-5-yl-carboxamido)-äthyl⟩-benzolsulfonyl)-carbamidsäuremethylester (Schmp. 214°C, hergestellt durch Umsetzung des 4-(2-⟨6-Oxo-5H-thieno-⟨2,3-c⟩-pyrrol-5-yl-carboxamido⟩-äthyl)-benzolsulfonamid mit Chlorameisensäuremethylester) werden in 50 ml Dioxan gelöst. Nach Zusatz von 0,7 ml Cyclohexylamin wird drei Stunden unter Rühren auf 100°C erhitzt und dabei das entstehende Methanol abdestilliert. Nach dem Abdestillieren des Lösungsmittels nimmt man in verdünnter Salzsäure auf und saugt ab. Nach dem Umfällen aus verdünnter Ammoniaklösung mit verdünnter Salzsäure wird der N-(4-⟨2-(6-Oxo-5H-thieno-⟨2,3-c⟩-pyrrol-5-yl-carboxamido)-äthyl⟩-benzolsulfonyl)-N'-cyclohexyl-harnstoff aus Äthanol-Dimethylformamid umkristallisiert und schmilzt bei 238 -239°C.

In analoger Weise erhält man den

N-(4-⟨2-(6-Oxo-5H-thieno-⟨2,3-c⟩-pyrrol-5-yl-carboxamido)-äthyl⟩-benzolsulfonyl)-N'-(4-chlor-cyclohexyl)-harnstoff
vom Schmp. 195 - 198°C (aus Äthanol)

Patentansprüche:

1. Sulfonylharnstoffe der Formel

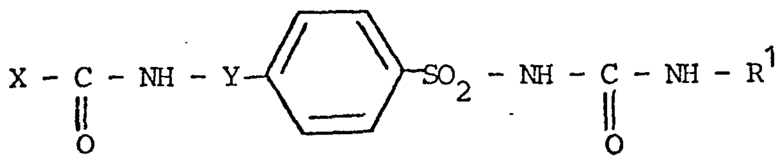

in welcher bedeuten:

X

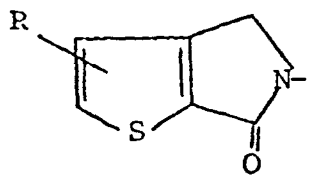 , 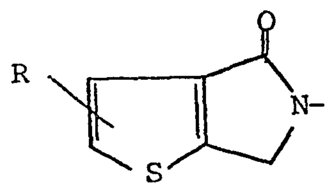 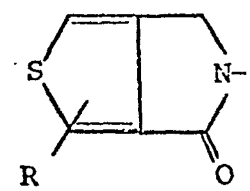

worin R Wasserstoff oder Halogen ist

Y Alkylen mit 2 bis 3 C-Atomen,

$R^1$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl,

und deren physiologisch verträgliche Salze.

2. Verfahren zur Herstellung von Sulfonylharnstoffen gemäß Anspruch 1, dadurch gekennzeichnet, daß man

a) mit der Gruppe

X - CO - NH - Y -

in 4-Stellung substituierte Benzolsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

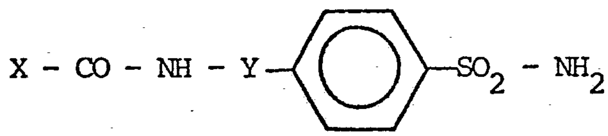

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte Benzolsulfonyl-isoharnstoffäther, -isothioharnstoffäther, - parabansäuren oder -halogenameisensäureamidine spaltet,

c) in

X - CO - NH - Y -

substituierten Benzolsulfonylthioharnstoffen das Schwefelatom durch Sauerstoff ersetzt,

d) in Benzolsulfonylharnstoffe. der Formel

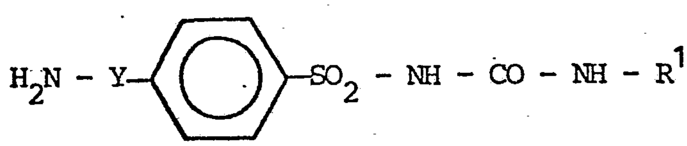

gegebenenfalls stufenweise den Rest

X - CO -

einführt,

und die Reaktionsprodukte gegebenenfalls zur Salzbildung mit alkalischen Mitteln behandelt.

3. Arzneimittel auf Basis eines Sulfonylharnstoffs gemäß Anspruch 1 oder eines seiner Salze.

4. Verwendung eines Sulfonylharnstoffs gemäß Anspruch 1 oder eines seiner Salze bei der Bekämpfung von Diabetes.

5. Verfahren zur Herstellung eines Arzneimittels gemäß Anspruch 3, dadurch gekennzeichnet, daß man einen Sulfonylharnstoff der im Anspruch 1 angegebenen Formel oder eines seiner Salze in eine geeignete Applikationsform bringt.

6. Verfahren zur Senkung des Blutzuckerspiegels bei der Behandlung des Diabetes, dadurch gekennzeichnet, daß man eine wirksame Menge einer Verbindung gemäß Anspruch 1 verabreicht.

Patentanspruch für Österreich:

1. Verfahren zur Herstellung von Sulfonylharnstoffen der Formel

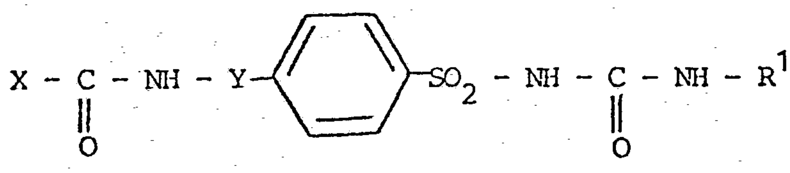

in welcher bedeuten:

X

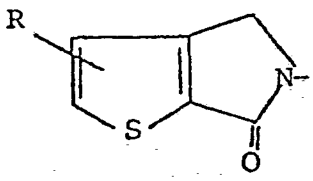

,

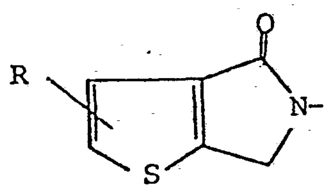

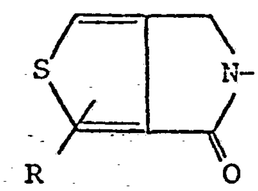

worin R Wasserstoff oder Halogen ist

Y Alkylen mit 2 bis 3 C-Atomen,

$R^1$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl,

und von deren physiologisch verträglichen Salzen, dadurch gekennzeichnet, daß man

a) mit der Gruppe

$$X - CO - NH - Y -$$

in 4-Stellung substituierte Benzolsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

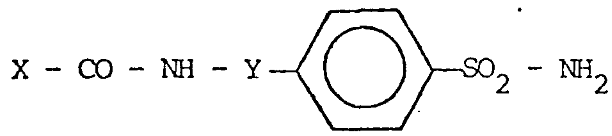

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte Benzolsulfonyl-isoharnstoffäther, -isothioharnstoffäther, - parabansäuren oder -halogenameisensäureamidine spaltet,

c) in

$$X - CO - NH - Y -$$

substituierten Benzolsulfonylthioharnstöffen das Schwefelatom durch Sauerstoff ersetzt,

d) in Benzolsulfonylharnstoffe der Formel

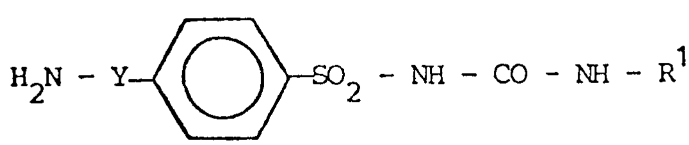

gegebenenfalls stufenweise den Rest

$$X - CO -$$

einführt,

und die Reaktionsprodukte gegebenenfalls zur Salzbildung mit alkalischen Mitteln behandelt.

0031088

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 80107818.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | AT - B - 276 423 (FARBWERKE HOECHST)<br>+ Ansprüche; Seite 4, Zeilen 14-28 +<br>-- | 1-3, 5 | C 07 D 495/04<br>A 61 K 31/64//<br>(C 07 D 495/04<br>C 07 D 209/00<br>C 07 D 333/00) |
| | DE - A - 2 256 979 (FARBWERKE HOECHST)<br>+ Seite 7; Ansprüche +<br>-- | 1-3,5 | |
| | DE - A1 - 2 621 958 (HOECHST AG)<br>+ Ansprüche; Seite 10, Zeile 10 bis Seite 15 +<br>---- | 1-3,5 | |
| | | | RECHERCHIERTE SACHGEBIETE (Int. Cl.³) |
| | | | C 07 C 143/00<br>C 07 D 209/00<br>C 07 D 213/00<br>A 61 K 31/00<br>C 07 D 495/00 |

**UNVOLLSTÄNDIGE RECHERCHE**

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5

Unvollständig recherchierte Patentansprüche:

Nicht recherchierte Patentansprüche: 4,6

Grund für die Beschränkung der Recherche:

Verfahren zur therapeutischen Behandlung des menschlichen oder tierischen Körpers gem. Art. 52 (4) EPÜ

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 11-03-1981 | TENGLER |